# EUROPÄISCHE PATENTANMELDUNG

(11) **EP 0 692 478 A1**
(43) Veröffentlichungstag der Anmeldung: **17.01.1996**
(21) Anmeldenummer: 95110766.3
(22) Anmeldetag: 11.07.1995
(51) Int. Cl.: C07D 263/22, C07C 215/08

(54) **Verfahren zur Herstellung optisch aktiver, 4-substituierter (S)-2-Oxazolidinone, neue (S)-2-Oxazolidinone, neue optisch aktive (S)Aminoalkohole sowie Verwendung dieser Verbindungen**

(30) Priorität: 15.07.1994 DE 4425067
(71) Anmelder: Degussa Aktiengesellschaft, D-60311 Frankfurt (DE)
(72) Erfinder: Bommarius, Andreas, Dr., D-60323 Frankfurt (DE); Drauz, Karlheinz, Prof. Dr., D-63579 Freigericht (DE); Krix, Georg, D-50679 Köln (DE); Kula, Maria-Regina, Prof. Dr., D-52382 Niederziehr (DE); Schwarm, Michael, Dr., D-63755 Alzenau (DE)

(57) **Zusammenfassung**

2.1. Verfahren zur Herstellung optisch aktiver, 4-substituierter (S)-2-Oxazolidinone der allgemeinen Formel (IV) 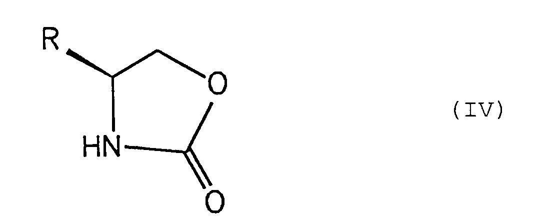
worin R für eine raumerfüllende, verzweigte, wenigstens ein tertiäres C-Atom enthaltende Alkylgruppe mit 5 - 10 C-Atomen steht, sind bislang nicht bekannt geworden.

2.2. Dadurch, daß ein optisch aktiver (S)-Aminoalkohol der allgemeinen Formel (III) 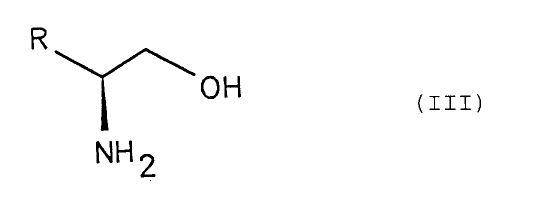
worin R für eine raumerfüllende, verzweigte, wenigstens ein tertiäres C-Atom enthaltende Alkylgruppe mit 5 - 10 C-Atomen steht,
mit einem Chlorameisensäureester am Stickstoff acyliert und das Zwischenprodukt mit einer katalytisch wirksamen Menge einer Base zum entsprechenden (S)-2-Oxazolidinon der Formel (IV) cyclisiert wird, gelingt die Darstellung der angestrebten Produkte in hoher Reinheit und guter Ausbeute. Verbindungen der Formeln (III) und (IV) sind neu.

2.3. Pharmazeutische Zwischenprodukte,
asymmetrische Synthese.

## Beschreibung

Die Erfindung betrifft ein Verfahren zur Herstellung optisch aktiver 4-substituierter (S)-2-Oxazolidinone der allgemeinen Formel (IV) 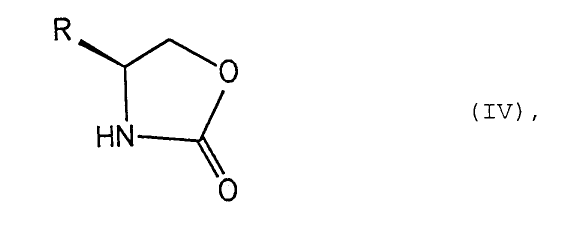
worin R für eine raumerfüllende, verzweigte, wenigstens ein tertiäres C-Atom enthaltende Alkylgruppe mit 5 - 10 C-Atomen steht, sowie neue, optisch aktive (S)-2-Oxazolidinone der allgemeinen Formel (IV), neue optisch aktive (S)-Aminoalkohole der allgemeinen Formel III 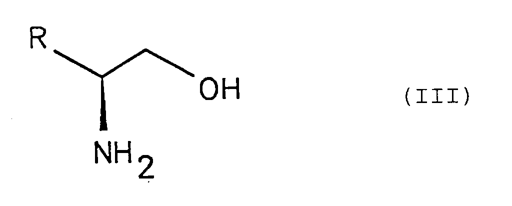
worin R für eine raumerfüllende, verzweigte, wenigstens ein tertiäres C-Atom enthaltende Alkylgruppe mit 5 - 10 C-Atomen steht, wobei die Verbindungen der Formel (III) als Zwischenprodukte für die Herstellung der Verbindungen der Formel (IV) von Interesse sind. Ferner beschreibt die Erfindung die Verwendung der neuen Verbindungen.

Optisch aktive (S)-2-Oxazolidinone und die bei deren Synthese als Zwischenprodukt in Erscheinung tretenden entsprechenden optisch aktiven Aminoalkohole sind äußerst wichtige Substanzklassen in der organischen Chemie. Sie finden breite Anwendung in der asymmetrischen Synthese, bei der Herstellung von pharmazeutischen Wirkstoffen wie zum Beispiel Peptiden, der Synthese von Insektiziden, der Spaltung racemischer Gemische und auf anderen Gebieten (weiterführende Literatur zu diesen Themen unter anderem in: J. Org. Chem. 1993, 58, 3568).

Der Einsatz von optisch aktiven Aminoalkoholen der allgemeinen Formel (III) in der asymmetrischen Synthese beruht sehr häufig darauf, daß Aminoalkohole mit sterisch anspruchsvollen Seitengruppen in geeignete Derivate eingebaut werden, mit denen eine asymmetrische Katalyse oder asymmetrische Induktion bei der Synthese von Folgeprodukten möglich ist. Beispiele für derartige Aminoalkohole sind unter anderem Phenylglycinol (R = Ph), Phenylalaninol (R = CH₂Ph), Valinol (R = CHMe₂) und tert.-Leucinol (R = CMe₃). Diese können beispielsweise zu optisch aktiven Oxazolinen und ähnlichen Verbindungen derivatisiert werden, die wiederum als Liganden für hochwirksame Katalysatoren eingesetzt werden können, zum Beispiel in der asymmetrischen Cyclopropanierung und Reduktion von Olefinen, der Hydrosilylierung und Reduktion von Ketonen, bei Diels-Alder-Reaktionen und nucleophilen Substitutionen (weiterführende Literatur z. B. in: Angew. Chem. 1991, 103, 556). Als Beispiele für in der asymmetrischen Synthese vielseitig einsetzbare Folgeprodukte der optisch aktiven Aminoalkohole seien 4-substituierte 2-Oxazolidinone (Org. Synth. 1989, 68, 77 und dort zitierte Lit.), bicyclische Lactame (Tetrahedron 1991, 47, 9503 und dort zitierte Lit.) und Formamidine (Tetrahedron 1992, 48, 2589 und dort zitierte Lit.) genannt.

In den genannten und vielen weiteren Fällen üben die Seitengruppen der eingebauten optisch aktiven Aminoalkohole der allgemeinen Formel (III) aus sterischen oder stereoelektronischen Gründen einen dirigierenden Einfluß auf die an und mit diesen Molekülen ablaufenden Reaktionen aus, woraus sich die teilweise außerordentlich hohen Enantio- oder Diastereoselektivitäten solcher Folgereaktionen ergeben. Dieser dirigierende Einfluß ist in vielen Fällen umso größer, je raumerfüllender die genannten Seitengruppen R sind (Angew. Chem. 1991, 103, 556). So ist der erzielte e.e. oder d.e. häufig größer, wenn die Seitengruppe ein tert.-Butylrest (R = CMe₃) anstelle eines Isopropylrestes (R = CHMe₂) ist (Beispiele siehe unter anderem: Tetrahedron Lett. 1990, 31, 6005; Tetrahedron 1992, 48, 2589; Angew. Chem. 1987, 99, 1197; J. Am. Chem. Soc. 1988, 110, 1238).

Angesichts dieser Tatsachen ist es Aufgabe der vorliegenden Erfindung, ein Verfahren zur Herstellung von Oxazolidinonen anzugeben, welches insbesondere solche Derivate mit raumerfüllenden Seitenketten zugänglich macht, welche den sterischen Anspruch einer tert.-Butylgruppe nach Möglichkeit noch übersteigen, um so bei asymmetrischen Synthesen einen noch größeren dirigierenden Einfluß auszuüben und damit die Enantio- und Diastereoselektivität bei diesen Reaktionen noch zu verbessern. Eine weitere Aufgabe der Erfindung ist die Bereitstellung neuer Oxazolidinon-Verbindungen sowie neuer optisch aktiver (S)-Aminoalkohole sowie deren Verwendung.

Diese und weitere nicht näher ausgeführte Probleme werden durch ein Verfahren mit den Merkmalen des kennzeichnenden Teils des Anspruches 1 gelöst. Vorteilhafte Verfahrensmodifikationen werden in den von Anspruch 1 abhängigen Verfahrensansprüchen unter Schutz gestellt. Neue Verbindungen sind Gegenstand der Ansprüche 17 und 18.

Dadurch, daß ein optisch aktiver (S)-Aminoalkohol der allgemeinen Formel (III) 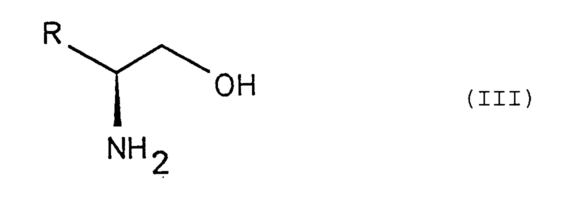
worin R für eine raumerfüllende, verzweigte, wenigstens ein tertiäres C-Atom enthaltende Alkylgruppe mit 5 - 10 C-Atomen steht, erfindungsgemäß in das entsprechende optisch aktive 4-substituierte (S)-2-Oxazolidinon der allgemeinen Formel (IV) 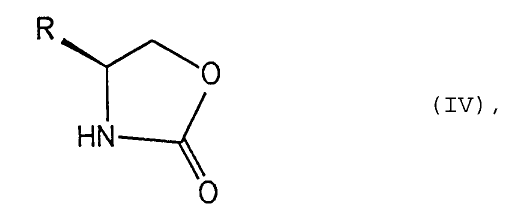
worin R die unter (III) angegebene Bedeutung hat, überführt wird, indem der Aminoalkohol durch Umsetzung mit einem Chlorameisensäureester unter pH-Kontrolle am Stickstoff acyliert wird und der intermediär gebildete N-geschützte Aminoalkohol dann basenkatalysiert zum (S)-2-Oxazolidinonen der allgemeinen Formel (IV) cyclisiert wird, gelingt es in einer schonenden Reaktion, die angestrebten sterisch besonders anspruchsvollen Verbindungen der Formel (IV) in guter Ausbeute bereitzustellen.

Die Acylierung wird im neutralen bis leicht basischen Bereich in einem 2-Phasensystem aus Wasser und einem organischen Lösungsmittel durchgeführt, bevorzugt in einem Bereich von pH 6 - 10, besonders bevorzugt pH 7 - 8,5. Als Chlorameisensäureester besonders bevorzugt sind Chlorameisensäuremethyl- und ethylester. Das organische Lösungsmittel muß die Reaktionspartner hinreichend gut lösen und unter den Reaktionsbedingungen inert sein, kann aber prinzipiell nahezu frei gewählt werden. Bevorzugt sind Lösungsmittel mit Etherstruktur oder Kohlenwasserstoffe. Besonders bevorzugt werden Lösungsmittel eingesetzt, die gleichzeitig für die Extraktion des Aminoalkohols der allgemeinen Formel (III) aus wäßriger Phase, die Acylierung mit Chlorameisensäureester, die darauf folgende basische Cyclisierung zum Oxazolidinon der allgemeinen Formel (IV) und dessen Kristallisation einsetzbar sind, da dann aufwendige und teure Lösungsmittelwechsel entfallen. Erfindungsgemäß ganz besonders bevorzugt für diesen Zweck sind Toluol und Xylole.

Die Cyclisierung kann mit einer Vielzahl von Basen durchgeführt werden, doch haben sich gemäß der vorliegenden Erfindung die einfachsten und billigsten Basen, nämlich Alkalimetallhydroxide als am besten geeignet herausgestellt. Ganz besonders bevorzugt ist Natriumhydroxid, das, wiederum bevorzugt, in fein granulierter oder gepulverter Form eingesetzt wird. Außerdem ist zum Erreichen einer vollständigen Cyclisierung erhöhte Temperatur von vorteilhaft wenigstens 50 °C bis zum Siedepunkt des verwendeten Lösungsmittels günstig. Der bei der Cyclisierung freiwerdende Alkohol destilliert aus dem Reaktionsgemisch ab, wenn die Reaktionstemperatur hinreichend hoch gewählt wird.

Wenn die Reaktion beendet ist, wird vorteilhaft die Base durch Zusatz einer äquivalenten Menge Säure neutralisiert, das Salz mit Wasser ausgewaschen und das Oxazolidinon der allgemeinen Formel (IV) durch Abkühlen, Einengen und gegebenenfalls Umkristallisation isoliert und gereinigt.

Die optisch aktiven (S)-Aminoalkohole der Formel (III) sind grundsätzlich aus optisch aktiven L-Aminosäuren der allgemeinen Formel (II) 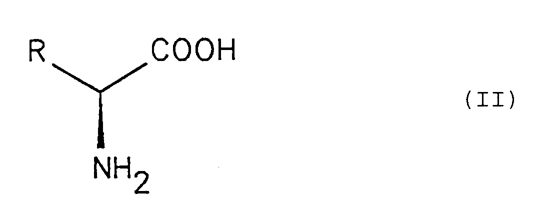
erhältlich. Bevorzugt werden Verbindungen der Formel (II) mit hydridischen Reagentien zu den (S)-Aminoalkoholen der allgemeinen Formel (III) reduziert. Als Reduktionsmittel kommen dabei Reagentien wie Lithiumaluminiumhydrid oder insbesondere mit einem Aktivator aktivierte Alkaliborhydride in Frage. Bei letzteren sind Lithium- und Natriumborhydrid bevorzugt, von denen wiederum letzteres aufgrund seines günstigen Preises besonders bevorzugt ist. Bezogen auf 1 Äquivalent Aminosäure, werden zur Reduktion 1,5 - 4, bevorzugt 2 - 2,5 Äquivalente des hydridischen Reduktionsmittels eingesetzt.

Als Aktivatoren kommen verschiedene Reagentien in Frage, so Bortrifluorid-Etherat, Trimethylchlorsilan, Iod, Chlor, Chlorwasserstoff oder Schwefelsäure (weiterführende Literatur zu derartigen Reduktionssystemen für Aminosäuren allgemein: J. Org. Chem. 1993, 58, 3568), von denen Iod und Schwefelsäure besonders bevorzugt sind. Bezogen auf 1 Äquivalent Alkaliborhydrid wird dabei bevorzugt 1/2 Äquivalent Iod oder Schwefelsäure zur Aktivierung eingesetzt.

Als Lösungsmittel sind solche mit Etherstruktur, insbesondere 1,2-Dimethoxyethan (DME) und Tetrahydrofuran (THF), besonders vorteilhaft, doch kommen prinzipiell auch andere Lösungsmittel wie Alkohole oder Acetale für die Reduktion in Frage. Diese kann innerhalb eines weiten Temperaturbereichs (ca. -20 °C - Siedetemperatur des verwendeten Lösungsmittels) durchgeführt werden, doch wird vorteilhaft so verfahren, daß zu einer Suspension von Natriumborhydrid und der Aminosäure der allgemeinen Formel (II) in einem geeigneten Lösungsmittel bei 0 - 30 °C eine Lösung des Aktivators in diesem oder einem anderen geeigneten Lösungsmittel getropft wird und danach zur Vervollständigung der Reaktion mehrere Stunden bis maximal zur Siedetemperatur des verwendeten Lösungsmittels erhitzt wird. Nach Abkühlung wird durch Zusatz von Alkohol, dann Wasser, dann Säure, bevorzugt Salzsäure, sauer gestellt, danach, bevorzugt mit Natronlauge, alkalisiert und mit einem geeigneten organischen Lösungsmittel, bei Bedarf unter Erwärmen, der optisch aktive Aminoalkohol der allgemeinen Formel (III) extrahiert. Er kann dann, wenn gewünscht, zur weiteren Reinigung destilliert, kristallisiert, chromatographiert oder in ein kristallines Salz, z. B. ein Hydrochlorid, überführt werden.

Die optisch aktiven L-Aminosäuren der allgemeinen Formel (II) sind erfindungsgemäß wiederum aus α-Ketocarbonsäuren der allgemeinen Formel (I) 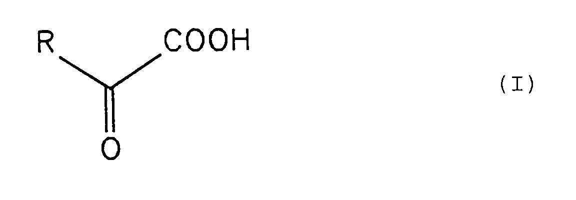
erhältlich, worin R jeweils für eine raumerfüllende, verzweigte, wenigstens ein tertiäres C-Atom enthaltende Alkylgruppe mit 5-10 C-Atomen steht. Bevorzugt geschieht dies durch co-faktorabhängige enzymatische reduktive Aminierung unter Verwendung von Dehydrogenasen.

Es war dabei insbesondere überraschend, keinesfalls vorhersehbar und erfindungsgemäß besonders vorteilhaft, daß die verwendeten Enzyme auch α-Ketosäuren der allgemeinen Formel (I) mit ihren besonders raumerfüllenden Resten R als Substrate zur Umwandlung in die L-Aminosäuren der allgemeinen Formel (II) akzeptieren und daß die Produkte auch noch mit hoher chemischer und Enantiomerenreinheit sowie in guter bis sehr guter Ausbeute erhalten werden.

Die Verbindungen der allgemeinen Formeln (III) und (IV) sind neu.

Verbindungen der allgemeinen Formel (II) sind neu, wenn R nicht Neopentyl ist (R = CH₂CMe₃).

Die Herstellung der in dieser Erfindung beschriebenen Verbindungen wird in folgendem Formelschema nochmals zusammengefaßt: 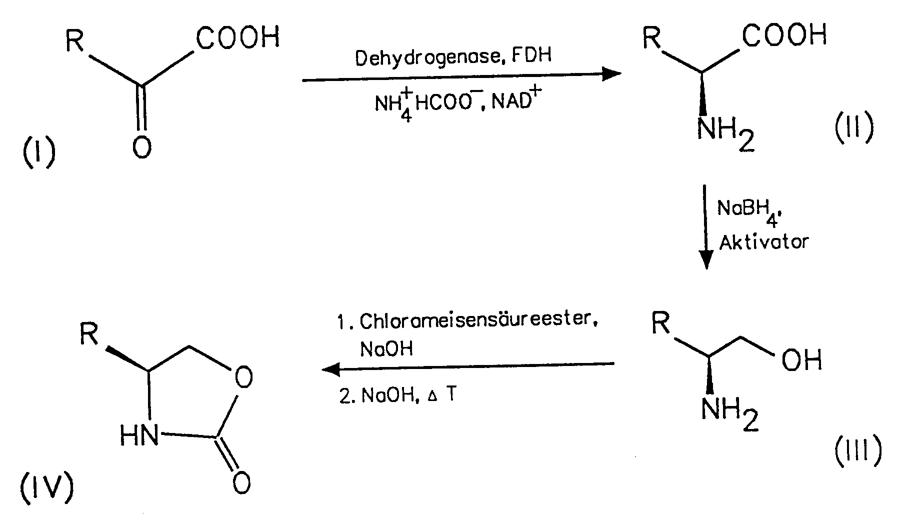
Insgesamt werden mit der vorliegenden Erfindung neue, optisch aktive L-Aminosäuren der allgemeinen Formel (II), (S)-Aminoalkohole der allgemeinen Formel (III) und 4-substituierte (S)-2-Oxazolidinone der allgemeinen Formel (IV) sowie Verfahren bereitgestellt, nach denen diese Verbindungen leicht, sicher, in guter bis sehr guter Ausbeute und in sehr hoher chemischer und insbesondere Enantiomerenreinheit herstellbar sind. Die so hergestellten Verbindungen finden Einsatz unter anderem in der Synthese von Pharmawirkstoffen und in der asymmetrischen Synthese.

Die erfindungsgemäßen Verbindungen sowie die Verfahren zu ihrer Herstellung werden durch die nachfolgenden Beispiele näher erläutert:

### Beispiel 1: (S)-Neopentylglycinol

Zu einer Suspension von 30,4 g (0,805 mol) Natriumborhydrid in 250 ml DME wurden 50,8 g (0,35 mol) L-Neopentylglycin gegeben. Anschließend wurde bei maximal 10 °C innerhalb von 2,5 h eine Lösung von 21,5 ml (0,4025 mol) Schwefelsäure in 75 ml DME zugetropft und dann 3 h auf 70 °C erhitzt. Nach Abkühlen wurden 60 ml MeOH zugesetzt. Anschließend wurde einrotiert, der Rückstand in 250 ml Wasser und 45 ml konz. Salzsäure aufgenommen und einige Zeit gerührt. Nach Zusatz von 300 ml Toluol wurde mit 65 ml 50 %iger Natronlauge basisch gestellt, auf 70 °C erwärmt, die organische Phase abgetrennt, filtriert, auf 90 g eingeengt und auf etwa 5 °C gekühlt. Nach Filtration, Waschen mit Toluol und Trocknen im Vakuumtrockenschrank wurden 40,0 g (87,1 %) (S)-Neopentylglycinol in Form farbloser Kristalle erhalten.

Die Struktur wurde durch ein NMR-Spektrum bestätigt.
[α]D20: +5,9° (c = 1, EtOH) Gehalt (Titration): > 99 %

| | | | | |
|---|---|---|---|---|
| C7H17NO 131,22 | Ber. | C 64,07 | H 13,06 | N 10,67 |
| | Gef. | C 64,01 | H 13,13 | N 10,90 |

### Beispiel 2: (S)-4-Neopentyl-2-oxazolidinon

130 g (0,99 mol) (S)-Neopentylglycinol wurden in 700 ml Toluol und 100 ml Wasser suspendiert. Bei 20 - 25 °C wurden innerhalb 1 h 99 ml (1,03 mol) Chlorameisensäureethylester zugetropft, wobei der pH-Wert mit 30 %iger Natronlauge bei etwa 8 gehalten wurde. Nach 30 min Nachrühren wurde auf 70 °C erwärmt und die Wasserphase abgetrennt. Die organische Phase wurde filtriert und durch azeotrope Destillation von restlichem Wasser befreit. Nach Zusatz von 2 g granuliertem Natriumhydroxid wurde langsam aufgeheizt. Bei 95 °C begann EtOH abzudestillieren, was bei 111 °C Sumpftemperatur beendet war. Nach Abkühlen auf 85 °C und Zusatz von 3 g Eisessig in 40 ml Wasser wurde kurz gerührt, die Wasserphase abgetrennt und die organische Phase nochmals mit 30 ml Wasser gewaschen. Nach Einengen auf 300 g und Kühlen über Nacht auf ca. 5 °C wurden nach Absaugen, Waschen und Trocknen 102,8 g (66,1 %) (S)-4-Neopentyl-2-oxazolidinon in Form farbloser Kristalle erhalten. Die Struktur wurde durch ein NMR-Spektrum bestätigt.
[α]D20: +9,3° (c = 1, EtOH)
Smp.:94-95°C

| | | | | |
|---|---|---|---|---|
| C8H15NO2 131,22 | Ber. | C 61,12 | H 9,62 | N 8,91 |
| | Gef. | C 61,25 | H 10,02 | N 8,99 |

Die Mutterlauge wurde eingedampft und der Rückstand aus 200 ml Hexan umkristallisiert, wodurch weitere 28,3 g (18,2 %) Produkt erhalten (Gesamtausbeute 84,3 %).

### Beispiel 3: Darstellung von (S)-Neopentylglycin

31.53 g (0.5 mol) Ammoniumformiat und 20.89 g (125 mmol) 2-Keto-4,4-Dimethylpentansäure Na-Salz werden in 400 ml Wasser suspendiert, der pH-Wert mit Ammoniak auf 8.2 eingestellt, so daß eine Lösung vorliegt und auf 500 ml aufgefüllt. Anschließend werden 71.7 mg (0.1 mmol) NAD⁺·H₂O-Cofaktor sowie 2000 U an Leucindehydrogenase (LeuDH) und 2500 U an Formiatdehydrogenase (FDH) zugegeben. Es wird eine Temperatur von 28 °C eingestellt, während der Reaktion gelinde gerührt und der pH-Wert auf 8.2 durch eine pH-Statisierungseinrichtung konstant gehalten. Nach 48 Stunden wird durch Bestimmung des Umsatzes per HPLC nachgewiesen, daß die Reaktion beendet ist. Die Enzyme werden über ein Ultrafilter der Porenweite von 10 kDa abgetrennt und die Reaktionslösung mit Ammoniak auf pH 9.5 eingestellt. Anschließend wird mit 2 % Aktivkohle geklärt und die fast farblose Lösung am Rotationsverdampfer eingeengt, die Aminosäure auskristallisiert, über eine Nutsche abgetrennt, dreimal mit wenig Ethanol gewaschen und im Vakuum über Nacht bei 50 °C getrocknet.
- Ausbeute:: 15.4 g (84.9 % der Theorie)
- Identitätsprüfung:: NMR-Spektrum
- Enantiomerenreinheit:: > 99.8 % e.e., gemessen durch chirale Gaschromatographie an Chirasil-Val

### Beispiel 4: Darstellung von (S)-3-Methyl-Isoleucin ((S))-3,3-Dimethyl-norvalin)

6.3 g (0.1 mol) Ammoniumformiat und 1.67 g (10 mmol) 2-Keto-3,3-Dimethylpentansäure Na-Salz werden in 80 ml Wasser suspendiert, der pH-Wert mit Ammoniak auf 8.2 eingestellt, so daß eine Lösung vorliegt und auf 100 ml aufgefüllt. Anschließend werden 14.34 mg (0.02 mmol) NAD⁺·3 H₂O-Cofaktor sowie 800 U an Leucindehydrogenase (LeuDH) und 500 U an Formiatdehydrogenase (FDH) zugegeben. Es wird eine Temperatur von 32 °C eingestellt, während der Reaktion gelinde gerührt und der pH-Wert auf 8.2 durch eine pH-Statisierungseinrichtung konstant gehalten. Nach spätestens 72 Stunden kann durch Bestimmung des Umsatzgrades per HPLC nachgewiesen werden, daß die Reaktion beendet ist. Die Reaktionslösung wird mit Ammoniak auf pH 9.5 eingestellt und anschließend mit 2 % Aktivkohle geklärt. Die fast farblose Lösung wird am Rotationsverdampfer eingeengt, die Aminosäure auskristallisiert, über eine Nutsche abgetrennt, dreimal mit wenig Ethanol gewaschen und im Vakuum über Nacht bei 50 °C getrocknet.
- Ausbeute:: 1.17 g (80.6 % der Theorie)
- Identitätsprüfung:: NMR-Spektrum
- Enantiomerenreinheit:: > 99.9 % e.e., gemessen durch chirale Gaschromatographie an Chirasil-Val

### Beispiel 5: Darstellung von (S)-Homoneopentylglycin ((S)-5,5-Dimethyl-norleucin)

Reaktion und Aufarbeitung wurden analog zu Beispiel 4 durchgeführt, mit der Ausnahme, daß während der gesamten Reaktionszeit eine Suspension vorlag und die Reaktion erst nach 96 Stunden beendet war.
- Einsatz:: 1.81 g (10 mmol) 2-Keto-5,5-Dimethyl-hexansäure Na-Salz
- Ausbeute:: 1.08 g (67.9 % der Theorie)
- Identitätsprüfung:: NMR-Spektrum
- Enantiomerenreinheit:: > 99.9 % e.e., gemessen durch chirale HPLC an Crownpak-CR+Säule

## Patentansprüche

1. Verfahren zur Herstellung optisch aktiver, 4-substituierter (S)-2-Oxazolidinone der allgemeinen Formel (IV) 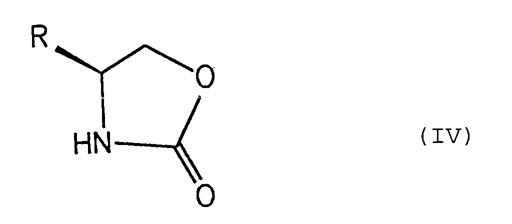 worin R für eine raumerfüllende, verzweigte, wenigstens ein tertiäres C-Atom enthaltende Alkylgruppe mit 5 - 10 C-Atomen steht,
**dadurch gekennzeichnet**,
daß ein optisch aktiver (S)-Aminoalkohol der allgemeinen Formel (III) 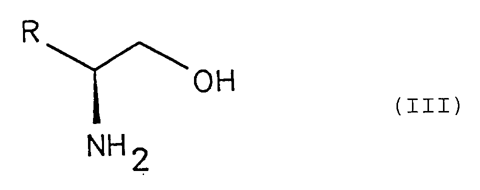 worin R für eine raumerfüllende, verzweigte, wenigstens ein tertiäres C-Atom enthaltende Alkylgruppe mit 5 - 10 C-Atomen steht,
mit einem Chlorameisensäureester am Stickstoff acyliert und das Zwischenprodukt mit einer katalytisch wirksamen Menge einer Base zum entsprechenden (S)-2-Oxazolidinon der Formel (IV) cyclisiert wird.

2. Verfahren nach Anspruch 1,
**dadurch gekennzeichnet**,
daß als Chlorameisensäureester Chlorameisensäureethyl- oder -methylester eingesetzt wird.

3. Verfahren nach einem der Ansprüche 1 oder 2,
**dadurch gekennzeichnet**,
daß die Acylierung in einem 2-Phasen-System aus Wasser und einem organischen Lösungsmittel unter pH-Kontrolle durchgeführt wird.

4. Verfahren nach Anspruch 1,
**dadurch gekennzeichnet**,
daß als Base Natriumhydroxid eingesetzt wird.

5. Verfahren nach einem der Ansprüche 1 bis 4,
**dadurch gekennzeichnet**,
daß die Cyclisierung bei einer Temperatur zwischen 50 °C und der Siedetemperatur des verwendeten organischen Lösungsmittels durchgeführt wird.

6. Verfahren nach Ansprüchen 1 - 5,
**dadurch gekennzeichnet**,
daß nach beendeter Cyclisierung die Base durch Zusatz einer äquivalenten Menge Säure neutralisiert, das Salz mit Wasser aus dem organischen Lösungsmittel ausgewaschen und das (S)-2-Oxazolidinon der Formel (IV) durch Abkühlen und Einengen isoliert und erforderlichenfalls durch Umkristallisation oder Chromatographie weiter gereinigt wird.

7. Verfahren gemäß einem der Ansprüche 3, 5 oder 6,
**dadurch gekennzeichnet**,
daß es sich bei dem organischen Lösungsmittel um Toluol handelt.

8. Verfahren nach einem der vorhergehenden Ansprüche,
**dadurch gekennzeichnet**,
daß der optisch aktive (S)-Aminoalkohol der allgemeinen Formel (III) durch Reduktion einer optisch aktiven L-Aminosäure der allgemeinen Formel 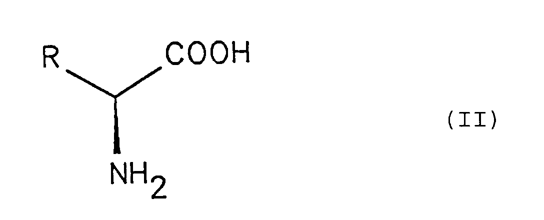 worin R für eine raumerfüllende, verzweigte, wenigstens ein tertiäres C-Atom enthaltende Alkylgruppe mit 5 - 10 C-Atomen steht, mit einem hydridischen Reduktionsmittel erhalten wird.

9. Verfahren nach Anspruch 8,
**dadurch gekennzeichnet**,
daß als hydridisches Reduktionsmittel Lithiumaluminiumhydrid eingesetzt wird.

10. Verfahren nach Anspruch 8,
**dadurch gekennzeichnet**,
daß als hydridisches Reduktionsmittel ein aktiviertes Alkaliborhydrid eingesetzt wird.

11. Verfahren nach Anspruch 10,
**dadurch gekennzeichnet**,
daß als Alkaliborhydrid Natrium- oder Lithiumborhydrid eingesetzt wird.

12. Verfahren nach einem der Ansprüche 10 oder 11,
**dadurch gekennzeichnet**,
daß als Aktivator Iod, Chlor, Chlorwasserstoff oder Schwefelsäure eingesetzt wird.

13. Verfahren nach einem der Ansprüche 8 - 12,
**dadurch gekennzeichnet,**
daß die Reaktion in einem Lösungsmittel, bevorzugt einem Ether, durchgeführt wird.

14. Verfahren nach einem der Ansprüche 8 - 13,
**dadurch gekennzeichnet**,
daß die Reaktion zwischen -20 °C und der Siedetemperatur des Lösungsmittels durchgeführt wird.

15. Verfahren nach einem der Ansprüche 8 - 14,
**dadurch gekennzeichnet**,
daß das Reaktionsgemisch nach beendeter Reduktion durch Zugabe von einem Alkohol, dann Wasser und dann einer Säure hydrolysiert wird, anschließend alkalisiert wird, der optisch aktive (S)-Aminoalkohol der allgemeinen Formel (III) mit einem organischen Lösungsmittel extrahiert, anschließend durch Eindampfen isoliert und erforderlichenfalls durch Chromatographie oder Destillation oder Umkristallisation oder Fällung als Hydrochlorid weiter gereinigt wird.

16. Verfahren nach einem der vorhergehenden Ansprüche,
**dadurch gekennzeichnet**,
daß zur Herstellung der optisch aktiven L-Aminosäure der allgemeinen Formel (II) eine α-Ketocarbonsäure der allgemeinen Formel (I) 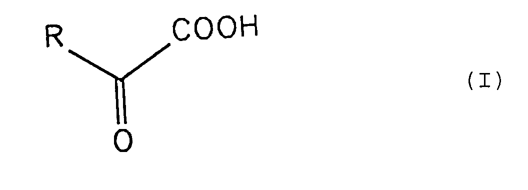 worin R für eine raumerfüllende, verzweigte, wenigstens ein tertiäres C-Atom enthaltende Alkylgruppe mit 5 - 10 C-Atomen steht, durch eine co-faktorabhängige enzymatische Reaktion unter Verwendung von Dehydrogenasen reduktiv aminiert wird.

17. Optisch aktive, 4-substituierte (S)-2-Oxazolidinone der allgemeinen Formel (IV) 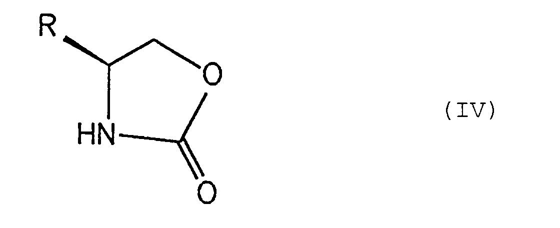 worin R für eine raumerfüllende, verzweigte, wenigstens ein tertiäres C-Atom enthaltende Alkylgruppe mit 5 - 10 C-Atomen steht.

18. Neue, optisch aktive (S)-Aminoalkohole der allgemeinen Formel (III) 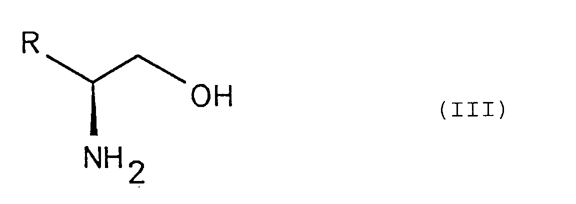 worin R für eine raumerfüllende, verzweigte, wenigstens ein tertiäres C-Atom enthaltende Alkylgruppe mit 5 - 10 C-Atomen steht.

19. Verwendung der neuen Verbindungen gemäß einem der Ansprüche 17 oder 18 zur Synthese von pharmazeutisch wirksamen Verbindungen.

20. Verwendung der neuen Verbindungen gemäß einem der Ansprüche 17 oder 18 zur Synthese von Liganden, Katalysatoren und Induktoren für die asymmetrische Synthese.
